(19) Europäisches Patentamt
European Patent Office
Office européen des brevets

(11) Veröffentlichungsnummer: **0 282 450**
**A2**

(12) **EUROPÄISCHE PATENTANMELDUNG**

(21) Anmeldenummer: **88810128.4**

(22) Anmeldetag: **02.03.88**

(51) Int. Cl.4: **C 07 C 79/36**
C 07 C 76/02

(30) Priorität: **10.03.87 CH 877/87**

(43) Veröffentlichungstag der Anmeldung:
**14.09.88 Patentblatt 88/37**

(84) Benannte Vertragsstaaten:
**CH DE ES FR GB IT LI**

(71) Anmelder: **CIBA-GEIGY AG**
**Klybeckstrasse 141**
**CH-4002 Basel (CH)**

(72) Erfinder: **Weis, Claus D., Dr**
**Hangelimattweg 112**
**CH-4148 Pfeffingen (CH)**

(54) **Verfahren zur Herstellung von 5-Nitro-1,4-naphthochinonen.**

(57) Verfahren zur Herstellung von 5-Nitro-1,4-naphthochinon der Formel

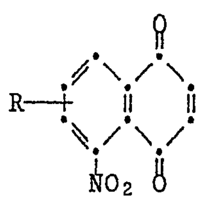

,

worin R Wasserstoff, $C_1$-$C_4$-Alkyl oder $C_1$-$C_4$-Alkoxy ist, durch Oxidation von 1-Nitronaphthalin oder entsprechend substituierter 1-Nitronaphthaline mit Silber-II-oxid.

EP 0 282 450 A2

**Beschreibung**

Verfahren zur Herstellung von 5-Nitro-1,4-naphthochinonen

Die Erfindung betrifft ein neues Verfahren zur Herstellung von 5-Nitro-1,4-naphthochinonen.

5-Nitro-1,4-naphthochinon, im folgenden kurz 5-Nitronaphthochinon genannt, ist ein wichtiges Farbstoffzwischenprodukt, das man mit Butadien nach Diels-Alder zum 5-Nitro-1,4,11,12-tetrahydroanthrachinon umsetzt (siehe z.B. N.N. Vorozhtsov et al. Zhur. Vsesoyuz. Khim. Obshchestva im D.I. Mendeleeva 5, 474 (1960) - Chem. Abstract Vol. 55, 1547e), welches anschliessend in das 1-Nitroanthrachinon, der Ausgangsverbindung für die Herstellung zahlreicher Dispersions-und Küpenfarbstoffe, überführt wird (siehe z.B. DE-A-24 50 883).

Es sind mehrere Verfahren zur Herstellung von 5-Nitronaphthochinon beschrieben. Nach den beiden wichtigsten Verfahrensvarianten geht man entweder aus von Naphthalin, das zunächst in 1-Stellung nitriert (Ullmann Bd. 17 (1979) Seite 392) und anschliessend oxidiert wird oder direkt vom 1,4-Naphthochinon, das man mit Nitriersäure in 5-Stellung nitriert (Ullmann, Bd. 17 (1979) Seite 121).

Aus ökonomischen und ökologischen Gründen ist man heute mehr und mehr daran interessiert, Oxidationsmittel zu verwenden, die sich am Ende der Reaktion leicht aus dem Reaktionsgemisch zurückgewinnen und regenerieren lassen, um anschliessend für weitere Oxidationsreaktionen wieder einsetzbar zu sein. So wird Naphthochinon beispielsweise durch Oxidation von Naphthalin mittels Cer-IV-sulfat erhalten, wobei man mit geschmolzenem Naphthalin arbeitet und das Cer-IV- sulfat aus dem während der Reaktion gebildeten Cer-III-sulfat mittels Elektrolyse oder Ozonolyse regeneriert (DE-A-32 20 305). Nach einem ähnlichen Verfahren gelingt auch die Herstellung von 5-Nitronaphthochinon aus 1-Nitronaphthalin (DE-A-23 01 803), wobei als Oxidationsmittel Ammoniumpersulfat verwendet wird in Kombination mit Cer-IV-sulfat und Silbernitrat. Durchgeführt wird das Verfahren in einem 2-Phasensystem mit wässriger und organischer Phase und das verbrauchte Persulfat wird durch Elektrolyse regeneriert.

Es wurde nun gefunden, dass sich Silber-II-oxid sehr gut als leicht elektrotechnisch regenerierbares Oxidationsmittel bei der Herstellung von 5-Nitro-1,4-naphthochinonen aus 1-Nitronaphthalinen eignet. Dieses Oxidationsmittel ist zudem kostengünstig, da sich das beim Oxidationsprozess gebildete Silber-I-salz beliebig oft reoxidieren und dann erneut als Silber-II-oxid verwenden lässt. Auch erzielt man mit diesem Oxidationsmittel eine gute Raum-Zeit-Ausbeute, da Silber-II-oxid ein hohes Redoxpotential verbunden mit einer spezifischen Oxidationswirkung vor allem bei Temperaturen unter 80°C besitzt und weil die Reaktanden in vergleichsweise hoher Konzentration umgesetzt werden können.

Gegenstand der vorliegenden Erfindung ist somit ein Verfahren zur Herstellung von 5-Nitro-1,4-naphthochinonen der Formel

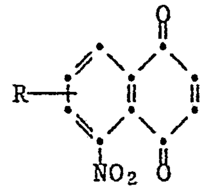

, .

worin R Wasserstoff, Alkyl mit 1 bis 4 C-Atomen oder Alkoxy mit 1 bis 4 C-Atomen ist, durch Oxidation von 1-Nitronaphthalin oder der entsprechend substituierten 1-Nitronaphthaline, dadurch gekennzeichnet, dass man 1-Nitronaphthalin oder ein entsprechend substituiertes 1-Nitronaphthalin in 30-60%iger Salpetersäure mit Silber-II-oxid bei Temperaturen von unter 80°C oxidiert und das Produkt isoliert.

Die als Ausgangsmaterial verwendeten 1-Nitronaphthaline sind bekannt und nach bekannten Methoden herstellbar.

Erfindungsgemäss kann eine Suspension des 1-Nitronaphthalins oder eines entsprechenden Derivats in 30-60%iger Salpetersäure vorgelegt werden, die dann mit Silber-II-oxid versetzt wird. Es ist aber auch möglich, eine Lösung des Silber-II-oxids in dieser Salpetersäure vorzulegen und das Naphthalinderivat hinzuzufügen. Die Reaktanden werden intensiv vermischt und bei Temperaturen von unter 80°C zur Reaktion gebracht. Ueberschreitet man 80°C Reaktionstemperatur, so nimmt nämlich die Ueberoxidation von 1-Nitronaphthalin zu Carbonsäuren in sehr starkem Masse zu. Vorzugsweise wird in einem Bereich von 0-50°C gearbeitet.

In einer bevorzugten Ausführungsform des erfindungsgemässen Verfahrens erfolgt die Umsetzung mit 38-48%iger wässriger Salpetersäure bei einer Reaktionstemperatur von 2-20°C.

Was das Verhältnis von Nitronaphthalin zu Silber-II-oxid anbetrifft, so verwendet man auf 1 Mol Nitronaphthalin zweckmässigerweise 1 bis 15 Mol, insbesondere 4 bis 10 Mol Silber-II-oxid. Geringere Mengen als 1 Mol Silber-II-oxid führen zu unrentabel kleinen Umsätzen.

Bevorzugt wird mit dem erfindungsgemässen Verfahren unsubstituiertes 5-Nitro-1,4-naphthochinon hergestellt.

Die gemäss vorliegendem Verfahren erhaltenen 5-Nitronaphthochinone werden auf übliche Weise aus dem Reaktionsgemisch isoliert. Beim Arbeiten ohne Lösungsmittel wird das Produkt zusammen mit nicht umgesetztem Ausgangsmaterial aus dem sauren Reaktionsgemisch mit Hilfe eines organischen Lösungsmittels extrahiert. Zur Extraktion verwendet man vor allem Ether, Benzol oder Benzolderivate, wie z.B. Chlorbenzole, Nitrobenzol, Xylol oder insbesondere Toluol. Man verwendet zweckmässigerweise ein Lösungsmittel, in dem das als Ausgangsmaterial eingesetzte Nitronaphthalin auch in der Kälte gut löslich ist, aus dem das gebildete Nitronaphthochinon jedoch beim Abkühlen ausfällt

und so durch einfache Kühlung isoliert werden kann. Das ausgefallene Produkt wird abfiltriert und getrocknet. Man erhält so 5-Nitronaphthochinon bzw. die entsprechenden Alkyl- oder Alkoxyderivate mit einer Reinheit von 90 bis 95 %. Das im Lösungsmittel verbliebene Nitronaphthalin kann zurückgeführt und in einem weiteren Oxidationszyklus als Ausgangsmaterial eingesetzt werden. Die wässrig anorganische Phase kann in eine Elektrolysezelle geleitet und das entstandene Silbernitrat elektrolytisch zu Silber-II-oxid regeneriert werden.

Die folgenden Beispiele dienen der Veranschaulichung der Erfindung; Teile bedeuten Gewichtsteile und Prozente Gewichtsprozente.

Beispiel 1:

1,73 g (0,01 mol) 1-Nitronaphthalin werden in 30 ml 40%iger wässriger Salpetersäure suspendiert und kräftig gerührt. Dann werden innerhalb 25 Minuten in kleinen Portionen 8,5 g (0,07 mol) Silber(II)oxid hinzugegeben. Nach jeder Zugabe wartet man bis Entfärbung der Lösung eintritt. Die Temperatur wird während der Zugabe von Silberoxid durch Aussenkühlung bei 5-12°C gehalten. Nach beendeter Reaktion rührt man noch weitere 5 Minuten, verdünnt mit 25 ml Wasser und extrahiert die Lösung zweimal mit je 30 ml Ether. Die Etherlösung wird mit Natriumbicarbonatlösung neutral gewaschen und über Natriumsulfat getrocknet. Es werden 2 g Rohprodukt erhalten, dessen Gehalt an 5-Nitronaphthochinon sich durch Gaschromatographie zu 52,2 % bestimmt. Umkristallisation aus Ethanol ergab gelbe Kristalle, 0,92 g (45 %), Schmelzpunkt: 165-166°C.

Die Wiedergewinnung von Silber-II-oxid erfolgt in einer Zelle wie in A.A.Noyes, D.DeVault, C.D.Coryell and T.J.Deahl, J. American Chem. Soc., 59, 1326 (1937) (besonders Seiten 1330 und 1331) beschrieben.

Dabei wird das salpetersaure Filtrat, das Silbernitrat enthält im Vakuum konzentriert, bis die Salpetersäurekonzentration 40-45 % beträgt. Dann wird die Lösung in eine Elektrolysezelle eingefüllt und bei 3 Ampere und 3,5 Volt während 4 Stunden oxidiert. Der schwarze Niederschlag wird abfiltriert und für zwei Stunden in heissem Wasser gerührt, filtriert und getrocknet.

Beispiel 2:

1,73 g (0,01 mol) 1-Nitronaphthalin werden in 30 ml 40%iger Salpetersäure suspendiert und wie in Beispiel 1 beschrieben mit 5 g (0,04 mol) Silber(II)oxid versetzt. Die Reaktionstemperatur beträgt 10°C. Die Aufarbeitung und die Gehaltsbestimmung erfolgt wie in Beispiel 1 und ergibt 41 % 5-Nitronaphthochinon.

## Patentansprüche

1. Verfahren zur Herstellung von 5-Nitro-1,4-naphthochinonen der Formel

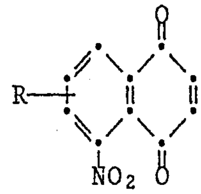

worin R Wasserstoff, $C_1$-$C_4$-Alkyl oder $C_1$-$C_4$-Alkoxy ist, durch Oxidation von 1-Nitronaphthalin oder einem entsprechend substituierten 1-Nitronaphthalin, dadurch gekennzeichnet, dass man 1-Nitronaphthalin oder ein entsprechend substituiertes 1-Nitronaphthalin in 30-60%iger Salpetersäure mit Silber-II-oxid bei Temperaturen von unter 80°C oxidiert und das Produkt isoliert.

2. Verfahren gemäss Anspruch 1, dadurch gekennzeichnet, dass man 35-50%ige wässrige Salpetersäure verwendet.

3. Verfahren gemäss Anspruch 1, dadurch gekennzeichnet, dass man auf 1 Mol Nitronaphthalin 1 bis 15 Mol Silber-II-oxid verwendet.

4. Verfahren gemäss Anspruch 2, dadurch gekennzeichnet, dass man die Umsetzung bei Temperaturen von 0-50°C durchführt.

5. Verfahren gemäss Anspruch 1, dadurch gekennzeichnet, dass man die Reaktion in 38-48%iger wässriger Salpetersäure bei einer Temperatur von 2 - 20°C durchführt.

6. Verfahren gemäss Anspruch 1, dadurch gekennzeichnet, dass das während der Reaktion entstehende Silbernitrat mittels Elektrolyse zu Silber-II-oxid reoxidiert und dieses erneut als Oxidationsmittel verwendet wird.

7. Die nach dem Verfahren gemäss Anspruch 1 erhaltenen 5-Nitro-1,4-naphthochinone.